## Europäisches Patentamt
## European Patent Office
## Office européen des brevets

(11) Veröffentlichungsnummer : **0 178 557**
**B1**

(12) **EUROPÄISCHE PATENTSCHRIFT**

(45) Veröffentlichungstag der Patentschrift :
25.05.88

(51) Int. Cl.⁴ : **C 07 C 139/00, C 07 C 143/18, C 14 C 9/02**

(21) Anmeldenummer : 85112631.8

(22) Anmeldetag : 04.10.85

(54) Verfahren zur Herstellung von Fettungsmittel für Leder und Pelze.

(30) Priorität : 12.10.84 DE 3437443

(43) Veröffentlichungstag der Anmeldung :
23.04.86 Patentblatt 86/17

(45) Bekanntmachung des Hinweises auf die Patenterteilung : 25.05.88 Patentblatt 88/21

(84) Benannte Vertragsstaaten :
AT BE CH DE FR GB IT LI NL

(56) Entgegenhaltungen :
EP-A- 0 130 753
EP-A- 0 153 036
DD-A- 220 338
DE-A- 3 309 049
DE-B- 1 016 700
DE-B- 1 246 717

(73) Patentinhaber : Henkel Kommanditgesellschaft auf Aktien
Postfach 1100 Henkelstrasse 67
D-4000 Düsseldorf-Holthausen (DE)

(72) Erfinder : Friese, Hans Herbert, Dr.
Schiesshecke 53
D-4019 Monheim (DE)
Erfinder : Pieper, Friedrich
Zehntenweg 10
D-4018 Langenfeld (DE)

## Beschreibung

Fettungsmittel für die Leder- und Pelzherstellung werden u. a. durch Einführung von Sulfonat- und/oder Sulfatgruppen in ungesättigt Öle und Fette hergestellt. Hierdurch erhält man wasserlösliche oder in Wasser emulgierbare Verbindungen, die ggf. zusammen mit weiteren gebräuchlichen Hilfsmitteln, z. B. Emulgatoren oder nicht sulfierten Ölen, in der Lickerflotte eingesetzt werden.

Die Herstellung derartiger Produkte kann durch Umsetzung von ungesättigte Anteile enthaltenden Fettstoffen mit Schwefelsäure, Oleum oder $SO_3$ erfolgen, wobei jedoch meist — besonders bei letzterem — dunkel gefärbte Produkte entstehen, wenn die Sulfierung nicht in Gegenwart großer Mengen eines indifferenten Lösungsmittels vorgenommen wird. Zur Erzielung möglichst reiner, salzarmer Produkte kann man die Sulfierung auch mittels eines $SO_3$-Luftgemisches vornehmen, doch ist auch hier eine nachfolgende Bleiche erforderlich (DE 12 46 717).

Die beschriebenen Schwierigkeiten bei der Sulfierung von ungesättigte Anteile enthaltenden Fettstoffen beruhen zum Teil darauf, daß die Viskosität während der Durchführung der Reaktion infolge der Bildung von Sulfonsäuren bzw. Schwefelsäureestern erheblich ansteigt. Hierdurch wird die weitere Umsetzung behindert, was zu langen Kontaktzeiten der Fettstoffe mit dem Sulfierungsmittel führt und Dunkelfärbungen bzw. Verkohlungen zur Folge hat. Außerdem wird dadurch der Durchsatz in vorhandenen Apparaturen stark vermindert. Insbesondere ist die Sulfierungsreaktion mit einem $SO_3$-Luftgemisch in Fallfilm- oder Kaskaden-Reaktoren durch die Viskositätserhöhung erheblich behindert, da die Fließgeschwindigkeit in den dünnen Flüssigkeitsschichten stark abnimmt, wobei es ggf. zum Verstopfen des Reaktors kommen kann. Sofern die Reaktion bei niedrigen Temperaturen durchgeführt wird, kommen Schwierigkeiten infolge von Inhomogenitäten der Fettstoffe noch hinzu.

Die Erfindung bezweckt eine Verbesserung der geschilderten Sulfierungsverfahren, insbesondere der Sulfierung mit einem $SO_3$-Luftgemisch. Gegenstand der Erfindung ist somit ein Verfahren zur Herstellung von Fettungsmitteln für Leder und Pelze durch Sulfierung von ungesättigte Anteile enthaltenden Fettstoffen mittels Schwefelsäure, Oleum oder $SO_3$ und nachfolgende Neutralisation sowie ggf. Bleiche, dadurch gekennzeichnet, daß man ein Gemisch aus (A) 80-20 Gew. - % eines $C_8$-$C_{24}$ Fettsäureglycerinesters mit einer Jodzahl $>$ 20 und (B) 20-80 Gew. - % eines bei Raumtemperatur flüssigen $C_8$-$C_{24}$ Fettsäureesters eine aliphatischen Monoalkohols der Kettenlänge $C_1$-$C_4$ mit einer Jodzahl von 50-100 der Sulfierung unterwirft.

Durch die beanspruchte Maßnahme wird eine bessere Homogenität der Fettstoffe und insbesondere eine niedrigere Viskosität der Sulfierungsprodukte erreicht, was eine bessere Steuerung des Reaktionsablaufes, höhere Durchsätze durch vorhandene Apparaturen, definiertere Sulfierungsgrade und die Herstellung homogener, blanker Sulfierungsprodukte von guter Farbqualität ermöglicht. Eine Abtrennung des zugesetzten Fettsäureesters nach der Sulfierung ist nicht erforderlich, da dieser ebenfalls sulfiert wird bzw. als Neutralölanteil zur Wirksamkeit des Fettungsmittels beiträgt. Falls sehr helle Produkte gewünscht werden, kann man nach der Sulfierung ohne weiteres mit $H_2O_2$ bleichen, ohne daß die sonstigen vorteilhaften Eigenschaften der Produkte beeinträchtigt werden. Die gute Homogenität und niedrige Viskosität der Sulfierungsprodukte führt darüber hinaus zu einer problemlosen Anwendung und einem verbesserten Eindringen des Fettungsmittels in das Leder.

Bei den Fettstoffen (Komponente (A) des Gemisches) handelt es sich um Fettsäureglycerinester des Kettenlängen $C_8$-$C_{24}$ mit Jodzahlen $>$ 20. Vorzugsweise kommen hier natürliche oder synthetisch hergestellte Triglyceride höherer Fettsäuren der Kettenlängen $C_{16}$-$C_{24}$ mit Jodzahlen von 50-200 in Betracht, insbesondere natürliche oder aus natürlichen pflanzlichen oder tierischen Fetten hergestellte Fettsäuretriglyceridgemische, deren Fettsäuren zum Teil aus einfach oder mehrfach ungesättigten Fettsäuren bestehen, z. B. Lardöl, Klauenöl, Fischöl, Rüböl, Erdnußöl oder Olivenöl. Die Komponente (A) hat einen Anteil von 80-20, vorzugsweise 70-50 Gew. - % des Gemisches.

Die Fettsäureester (Komponente (B) des Gemisches) leiten sich von Fettsäuren der Kettenlängen $C_8$-$C_{24}$ ab und besitzen Jodzahlen von 50-100. Als Alkoholkomponente für die Fettsäureester kommen aliphatische Monoalkohole der Kettenlängen $C_1$-$C_4$ wie beispielsweise Methanol, Ethanol, Isopropanol und Isobutanol, oder deren Mischungen in Frage. Die Ester sollen bei Raumtemperatur flüssig und homogen sein, d. h., sie besitzen Schmelzpunkte von $<$ 20 °C. Bevorzugt werden Fettsäureestergemische der Kettenlängen $C_{12}$-$C_{20}$ mit Jodzahlen von 60-80 eingesetzt, wobei die Alkoholkomponente des Esters Methanol, Ethanol und/oder Isopropanol ist, z. B. ein $C_{12}$-$C_{18}$ Fettsäuremethylester der Jodzahlen 60-70 oder ein $C_{16}$-$C_{18}$ Fettsäureethylester der Jodzahlen 60-70. Die Komponente (B) hat einen Anteil von 20-80, vorzugsweise 30-50 Gew. - % des Gemisches.

Die Sulfierung kann in an sich üblicher Weise mittels Schwefelsäure oder Oleum durchgeführt werden, wobei 100 Gewichtsteile des Gemisches, bestehend aus 80-20 Gew. - % (A) und 20-80 Gew. - % (B), vorzugsweise bestehend aus 70-50 Gew. - % (A) und 30-50 Gew. - % (B) mit 10-20 Gewichtsteilen des Sulfierungsmittels unter Rühren und guter Kühlung umgesetzt werden. Die Reaktionsprodukte sind homogen und bis zur Beendigung der Umsetzung gut rührbar. Sie werden nach Auswaschen des Sulfierungsmittelüberschusses mit Alkalien oder Ammoniak neutralisiert und ggf. mit $H_2O_2$ gebleicht.

Besondere Vorteile ergeben sich, wenn man die Sulfierung mittels eines $SO_3$-Luftgemisches mit bis zu 8 Vol. % $SO_3$, z. B. im Verhältnis $SO_3$ : Luft wie 3,5 : 96,5, in einem Fallfilm- oder Kaskaden-Reaktor in kontinuierlicher Reaktion bei 20-50 °C, gegebenenfalls unter Abführung der Reaktionswärme bis zu einem Gehalt von 3-30 Gew. - % an organisch gebundenem Schwefeltrioxid durchführt. Hierbei bleiden die Fließfähigkeit und Homogenität des Reaktionsproduktes bis zur Beendigung des Sulfierungsvorgangs erhalten, so daß es auch bei solchen Fettstoffen, die relativ langkettige gesättigte oder hoch ungesättigte Anteile enthalten, nicht zu einem Zusetzen des Reaktors oder zu Schwarzfärbungen bzw. Verkohlungen infolge zu langer Kontaktzeiten des Ölgemisches mit dem SO$_3$-Luftgemisch kommt.

Besonders bevorzugt werden Sulfierungen mittels eines SO$_3$-Luftgemisches mit 3-5 Vol. % SO$_3$ in einem Fallfilmreaktor bei 20-50 °C, gegebenenfalls unter Abführung des Reaktionswärme bis zu einem Gehalt von 5-15 Gew.-% an organisch gebundenem Schwefeltrioxid durchgeführt.

Die Reaktion wird vorzugsweise bei 20-50 °C durchgeführt, wobei unter guter Kühlung blanke, relativ hellfarbige Sulfierungsprodukte bei hoher Raum-Zeit-Ausbeute des Reaktors erhalten werden. Nach Beendigung der Reaktion werden die Sulfierungsprodukte mit einem Alkalihydroxid, einer Ammoniaklösung oder einem Amin neutralisiert und ggf. mit H$_2$O$_2$ gebleicht. Der angestrebte Sulfierungsgrad der Produkte richtet sich nach deren Verwendungszweck und liegt vorzugsweise zwischen 3 und 30 Gew.- %, besonders bevorzugt zwischen 5 und 15 Gew. - % an organisch gebundenem Schwefeltrioxid.

Die nach dem beanspruchten Verfahren erhaltenen Produkte stellen problemlos anzuwendende Leder- und Pelzfettungsmittel dar. Sie werden in üblicher Weise in Form wäßriger Emulsionen zum Lickern von leder in einer Menge von 3 bis 15 Gew. - % AS, bezogen auf das Falzgewicht des Leders, oder zur Pelzbehandlung angewendet. Die Produkte sind selbstemulgierend, so daß weitere Zusätze an Emulgatoren im allgemeinen nicht notwendig sind. Zur Erzielung spezieller Effekte können sie mit anderen üblichen Lederbehandlungsmitteln kombiniert werden, wie z. B. nicht sulfierte Öle oder Fette, wie z. B. Fischtran, Spermöl, Klauenöl, synthetische Fettungsmittel, wie Chlorparaffine, Paraffinsulfonate, Mineralöle oder dergleichen, oder sulfitierte Öle, wie Fischölsulfit, ggf. in Verbindung mit anionischen, nichtionischen oder kationischen Emulgatoren, z. B. Ethylenoxidanlagerungsprodukte an höhere Fettalkohole, Alkylphenole oder Fettamine.

## Beispiele

1. 60 Gew. - % Lardöl mit einer Jodzahl von 72, das bei Raumtemperatur ca. 10 Vol. % Festanteile abscheidet, werden bei 60 °C mit 40 Gew. - % eines C$_{10}$-C$_{20}$ Fettsäuremethylesters mit einer Jodzahl von 70 zu einem homogenen, blanken Öl gemischt. Anschließend wird die Mischung in einem Fallfilm-Reaktor mit einem SO$_3$-Luftgemisch im Verhältnis 4,5 : 95,5 bei 25-30 °C unter üblichen Reaktorbedingungen kontinuierlich sulfiert. Der SO$_3$-Gehalt nach Epton beträgt im sauren Ester ca. 6 %. Nach Neutralisation des sauren Esters mit konzentriertem Ammoniak bei einer Temperatur von 70 °C erfolgt bei 50 °C eine Bleiche mit 1 % einer 30-35 %igen H$_2$O$_2$-Lösung innerhalb von 2 Stunden. Anschließend wird der pH des Produktes auf 8,0 eingestellt. Es wird ein bei Raumtemperatur helles, rotbraunes, flüssiges Produkt mit einem Wassergehalt von ca. 22 % erhalten, das sich in Wasser leicht emulgieren läßt.

2. Eine Mischung aus 60 Gew. - % Lardöl mit einer Jodzahl von 72, das bei Raumtemperatur ca. 10 Vol. % Festanteile abscheidet, und 40 Gew. - % eines C$_{12}$-C$_{20}$ Fettsäureisopropylesters mit einer Jodzahl von 68 wird in einem Rührbehälter mit 18 Gew. - % Oleum (20 % SO$_3$-Gehalt), bezogen auf die Ölmenge, bei 30-33 °C in üblicher Weise sulfiert und gewaschen. Der SO$_3$-Gehalt nach Epton im sauren Ester beträgt ca. 6 %. Anschließend wird der saure Ester bei 60 °C mit konzentriertem Ammoniak auf einem pH von 8,0 neutralisiert. Man erhält ein blankes, rotbraunes Produkt mit einem Wassergehalt von ca. 23 %, das sich in Wasser leicht emulgieren läßt.

3. Eine Mischung aus 50 Gew. - % Fischöl mit einer Jodzahl von 155 und 50 Gew. - % C$_{16}$-C$_{18}$ Fettsäuremethylester mit einer Jodzahl von 75 wird in einem Fallfilm-Reaktor mittels eines SO$_3$-Luftgemisches im Verhältnis 4,5 : 95,5 bei 40-45 °C unter üblichen Reaktorbedingungen kontinuierlich auf einem SO$_3$-Gehalt nach Epton von 5,8 % im sauren Ester sulfiert. Nach Neutralisation des sauren Esters mit Ammoniak bei einer Temperatur von 70 °C erfolgt eine Bleiche bei 50 °C mit 1 % einer ca. 30 %igen Wasserstoffperoxidlösung innerhalb von 3 Stunden. Anschließend wird der pH mit konzentriertem Ammoniak auf einem pH von 8,0 eingestellt. Es wird ein bei Raumtemperatur flüssiges, rotbraunes Öl erhalten mit einem Wassergehalt von 25 %, das sich in Wasser leicht emulgieren läßt.

4. Herstellung von Möbelleder

Versuchsmaterial : Wet-blue, gefalzt

| Waschen: | 200 | Gew.-% | Wasser 50 °C | 10 Minuten |
|---|---|---|---|---|
| | | | Flotte ab | |
| | | | neue Flotte | |

(Fortsetzung)

| Nachgerbung: | 100 | " | % | Wasser 50 $^{o}$C | |
| | 3 | " | % | Chromgerbstoff 33 % basisch | |
| | 3 | " | % | amphoterer synthetischer Gerbstoff | 60 Minuten |
| | 1,5 | " | % | Na-Al-Silikat | |
| | | | | spülen 40 $^{o}$C | |
| | | | | neue Flotte | |
| Neutralisation: | 100 | " | % | Wasser 40 $^{o}$C | |
| | 0,5 | " | % | maskierendes Neutralisationsmittel | |
| | 2 | " | % | Na-Bicarbonat | 60 Minuten |
| | | | | waschen bei 60 $^{o}$C | |
| Färbung: | 100 | " | % | Wasser 60 $^{o}$C | |
| | 2 | " | % | neutraler Hilfsgerbstoff | 10 Minuten |
| | 0,5 | " | % | Ammoniak | |
| | 3 | " | % | Farbstoff | 45 Minuten |
| Fettung: | 8 | " | % | AS Fettungsmittel nach Beispiel 1 | 1 : 2 mit Wasser emulgieren |
| | 4 | " | % | AS sulfitiertes Fettungsmittel | |
| | 1,5 | " | % | AS Fettalkoholsulfat $C_{12}$-$C_{18}$ | 45 Minuten |
| | 1,5 | " | % | Ameisensäure | 15 Minuten |
| | 1,5 | " | % | Ameisensäure | 30 Minuten |
| | | | | kalt spülen, | |
| | | | | Leder auf Bock, | |
| | | | | normal weiterarbeiten. ... | |

## 5. Herstellung von Schafbekleidungsnappa

Versuchsmaterial : Wet-blue-Schaffelle Gew. - % bezogen auf Falzgewicht

| Waschen: | 200 | Gew.-% | | Wasser 40 $^{o}$C | 20 Minuten |
| | 1 | " | % | anionisches Tensid | |
| | | | | neue Flotte | |
| Neutralisation: | 200 | " | % | Wasser 40 $^{o}$C | |
| | 2 | " | % | maskierendes Neutralisationsmittel | 45 Minuten |
| | | | | spülen bei 50 $^{o}$C | |

(Fortsetzung)

| Gerbung und Färbung: | 200 | " % | Wasser 50 °C | |
| | 2 | " % | neutraler Hilfsgerbstoff | 10 Minuten |
| | 1 | " % | Ammoniak | |
| | 3 | " % | Farbstoff | 50 Minuten |
| | 1 | " % | Ameisensäure | 30 Minuten |
| | 4 | " % | Harzgerbstoff | 20 Minuten |
| | 4 | " % | synthetischer Gerbstoff | 45 Minuten |
| Fettung: | 6 | " % | Fettungsmittel nach Beispiel 3 | 45 Minuten |
| | 0,5 | " % | AS komplexaktiver Emulgator | |
| | 0,5 | " % | Ameisensäure | 30 Minuten |
| | | | kalt spülen, hängetrocknen. | |

## 6. Herstellung von Oberleder

Versuchsmaterial : Wet-blue-Rindhaut Gew. - % bezogen auf Falzgewicht

| Waschen: | 200 | Gew.-% | Wasser 40 °C | 10 Minuten |
| | | | Flotte ab | |
| | | | neue Flotte | |
| Neutralisation: | 100 | " % | Wasser 40 °C | |
| | 1 | " % | Na-Formiat | 30 Minuten |
| | | | spülen bei 60 °C | |
| | | | neue Flotte | |
| Nachgerbung: | 100 | " % | Wasser 60 °C | |
| | 1 | " % | Farbstoff | 15 Minuten |
| | 2 | " % | AS Acrylatgerbstoff | 15 Minuten |
| | 4 | " % | synthetischer Gerbstoff | 45 Minuten |
| Fettung: | 4 | " % | AS Fettungsmittel nach Beispiel 1 | 1 : 2 mit Wasser emulgieren |
| | 2 | " % | AS Chlorparaffinsulfonat | |
| | 0,5 | " % | AS komplexaktiver Emulgator | 45 Minuten |
| | 0,5 | " % | Ameisensäure | 15 Minuten |
| | | | Leder auf Bock, normal trocknen und weiterarbeiten. | |

**0 178 557**

## Patentansprüche

1. Verfahren zur Herstellung von Fettungsmitteln für Leder und Pelze durch Sulfierung von ungesättigte Anteile enthaltenden Fettstoffen mittels Schwefelsäure, Oleum oder $SO_3$ und nachfolgender Neutralisation sowie ggf. Bleiche, dadurch gekennzeichnet, daß man ein Gemisch aus (A) 80-20 Gew. - % eines $C_8$-$C_{24}$ Fettsäureglycerinesters mit einer Jodzahl $>$ 20 und (B) 20-80 Gew. - % eines bei Raumtemperatur flüssigen $C_8$-$C_{24}$ Fettsäureesters eines aliphatischen Monoalkohols der Kettenlängen $C_1$-$C_4$ mit einer Jodzahl von 50-100 der Sulfierung unterwirft.

2. Verfahren nach Anspruch 1, dadurch gekennzeichnet, daß die Komponente (A) ein natürliches oder synthetisch hergestelltes $C_{16}$-$C_{24}$ Fettsäuretriglycerid mit einer Jodzahl von 50-200 ist.

3. Verfahren nach Ansprüchen 1 und 2, dadurch gekennzeichnet, daß die Komponente (A) Lardöl, Klauenöl, Fischöl, Rüböl, Erdnußöl oder Olivenöl ist.

4. Verfahren nach Ansprüchen 1-3, dadurch gekennzeichnet, daß die Komponente (B) ein $C_{12}$-$C_{20}$-Fettsäureester mit einer Jodzahl von 60-80 ist, wobei die Alkoholkomponente sich von einem aliphatischen Alkohol der Kettenlängen $C_1$-$C_3$ ableitet.

5. Verfahren nach Ansprüchen 1-4, dadurch gekennzeichnet, daß das zu sulfierende Gemisch aus 70-50 Gew. - % der Komponente (A) und 30-50 Gew. - % der Komponente (B) besteht.

6. Verfahren nach Ansprüchen 1-5, dadurch gekennzeichnet, daß die Sulfierung mit einem $SO_3$-Luftgemisch mit bis zu 8 Vol. % $SO_3$ in einem Fallfim- oder Kaskaden-Reaktor bei Temperaturen zwischen 20 °C und 50 °C, ggf. unter Abführung der Reaktionswärme, bis zu einem Gehalt von 3-30 Gew. - % an organisch gebundenem Schwefeltrioxid durchgeführt wird.

7. Verfahren nach Ansprüchen 1-6, dadurch gekennzeichnet, daß die Sulfierung mit einem $SO_3$-Luftgemisch mit 3-5 Vol. % $SO_3$ in einem Fallfilm-Reaktor bei Temperaturen zwischen 20 °C und 50°C, ggf. unter Abführung der Reaktionswärme, bis zu einem Gehalt von 5-15 Gew. - % an organisch gebundenem Schwefeltrioxid durchgeführt wird.

8. Anwendung der nach Ansprüchen 1-7 hergestellten Fettungsmittel für die Fettung von Leder und Pelzen in Form wäßriger Emulsionen zum Lickern von Leder bzw. zur Pelzbehandlung in einer Menge von 3-15 Gew. - % AS, bezogen auf das Falzgewicht der Leder oder Pelze.

9. Anwendung der nach Ansprüchen 1-7 hergestellten Fettungsmittel für die Behandlung von Leder und Pelzen entsprechend Anspruch 8 in Kombination mit nicht sulfierten natürlichen Ölen und Fetten oder synthetischen Fettungsmitteln auf Basis von Chlorparaffinen, Paraffinsulfonaten, Mineralölen und/oder sulfitierten Ölen, ggf. in Verbindung mit anionischen, nichtionischen oder kationischen Emulgatoren.

## Claims

1. A process for the production of oiling agents for leather and skins by sulfonating fats containing unsaturated fractions with sulfuric acid, oleum or $SO_3$, followed by neutralization and optionally by bleaching, characterized in that a mixture of (A) from 80 to 20 % by weight of a $C_8$-$C_{24}$ fatty acid glycerine ester having an iodine number above 20 and (B) from 20 to 80 % by weight of a $C_8$-$C_{24}$ fatty acid ester — liquid at room temperature. — of an aliphatic $C_1$-$C_4$ monoalcohol having an iodine number of from 50 to 100 is subjected to sulfonation.

2. A process as claimed in claim 1, characterized in that component (A) is a natural or synthetic $C_{16}$-$C_{24}$ fatty acid triglyceride having an iodine number of from 50 to 200.

3. A process as claimed in claims 1 and 2, characterized in that component (A) is lard oil, neat's-foot oil, fish oil, rape oil, peanut oil or olive oil.

4. A process as claimed in claims 1 to 3, characterized in that component (B) is a $C_{12}$-$C_{20}$ fatty acid ester having an iodine number of from 60 to 80, the alcohol component being derived from an aliphatic $C_1$-$C_3$ alcohol.

5. A process as claimed in claims 1 to 4, characterized in that from 70 to 50 % by weight of the mixture to be sulfonated consists of component (A) and from 30 to 50 % by weight of component (B).

6. A process as claimed in claims 1 to 5, characterized in that sulfonation is carried out with a mixture of $SO_3$ and air containing up to 8 % by volume of $SO_3$ in a falling-film or cascade reactor at temperatures of from 20 °C to 50 °C, optionally with dissipation of the heat of reaction, up to a content of from 3 to 30 % by weight of organically bound sulfur trioxide.

7. A process as claimed in claims 1 to 6, characterized in that sulfonation is carried out with a mixture of $SO_3$ and air containing 3 to 5 % by volume of $SO_3$ in a falling-film reactor at temperatures of from 20 °C to 50 °C, optionally with dissipation of the heat of reaction, up to a content of from 5 to 15 % by weight of organically bound sulfur trioxide.

8. The use of the oiling agents for oiling leather and skins produced by the process claimed in claims 1 to 7 in the form of aqueous emulsions for fat-liquoring leather and for treating skins in a quantity of from 3 to 15 % by weight of AS, based on the pared weight of the leather or skins.

9. The use of the oiling agents produced by the process claimed in claims 1 to 7 for treating leather and skins as claimed in claim 8 in combination with non-sulfonated naturel oils and fats or synthetic

**0 178 557**

stuffing agents based on chloroparaffins, paraffin sulfonates, mineral oils and/or sulfited oils, optionally in conjunction with anionic, nonionic or cationic emulsifiers.

**Revendications**

1. Procédé pour l'obtention d'agents de graissage pour cuir et peaux par sulfonation ou sulfuration de matières grasses contenant des fractions non saturées, à l'aide d'acide sulfurique, d'Oléum ou d'anhydride sulfurique et neutralisation subséquente ainsi que le cas échéant un blanchiment, caractérisé en ce qu'on soumet un mélange de (A) 80-20 % en poids d'un ester glycérique d'un acide gras en $C_8$-$C_{24}$ ayant un indice d'iode supérieur à 20 et (B) 20 à 80 % en poids d'un ester d'acide gras en $C_8$-$C_{24}$ avec un mono-alcool dont la longueur de chaîne est de $C_1$ à $C_4$ ayant un indice d'iode de 50 à 100, liquide à température ordinaire à la sulfonation.

2. Procédé selon la revendication 1, caractérisé en ce que le composant (A) est un triglycéride d'acide gras en $C_{16}$-$C_{24}$ obtenu synthétiquement ou naturel ayant un indice d'iode de 50 à 200.

3. Procédé selon les revendications 1 et 2, caractérisé en ce que le composant (A) est de l'huile de lard, de l'huile de pieds de bœuf, de l'huile de poisson, de l'huile de Colza, de l'huile d'Arachide ou de l'huile d'Olive.

4. Procédé selon les revendications 1 à 3, caractérisé en ce que le composant (B) est un ester d'acide gras en $C_{12}$-$C_{20}$ ayant un indice d'iode de 60 à 80, pour lequel la fraction alcool dérive d'un alcool alphatique dont la longueur de chaîne est en $C_1$-$C_3$.

5. Procédé selon les revendications 1 à 4, caractérisé en ce que le mélange à sulfoner consiste en 70 à 50 % en poids du composant (A) et de 30 à 50 % en poids du composant (B).

6. Procédé selon les revendications 1 à 5, caractérisé en ce que la sulfonation ou sulfuration est effectuée avec un mélange anhydride sulfurique/air ayant jusqu'à 8 % en volume d'anhydride sulfurique dans un réacteur en cascades ou à film tombant à des températures entre 20 °C et 50 °C éventuellement avec évacuation de la chaleur de la réaction, jusqu'à une teneur de 3 à 30 % en poids d'anhydride sulfurique lié organiquement.

7. Procédé selon les revendications 1 à 6, caractérisé en ce que la sulfonation ou sulfuration est effectuée avec un mélange anhydride sulfurique/air ayant de 3 à 5 % d'anhydride sulfurique en volume dans un réacteur à film tombant à des températures entre 20 et 50 °C, éventuellement avec évacuation de la chaleur de réaction jusqu'à une teneur de 5 à 15 % en poids d'anhydride sulfurique lié organiquement.

8. Application de l'agent de graissage obtenu selon les revendications 1 à 7 pour le graissage du cuir et des peaux sous la forme d'émulsions aqueuses pour le trempage du cuir ou pour le traitement des peaux en quantité de 3 à 15 % en poids (AS) rapporté au poids plissé du cuir ou des peaux.

9. Application de l'agent de graissage obtenu selon les revendications 1 à 7 pour le traitement du cuir et des peaux correspondant à la revendication 8 en combinaison avec des huiles naturelles non sulfonées et des graisses ou des agents de graissage synthétiques à base de paraffines chlorées, paraffines sulfonées, des huiles minérales, et/ou des huiles sulfonées le cas échéant en connexion avec des agents émulsionnants anioniques, non ioniques ou cationiques.

7